# EUROPÄISCHE PATENTSCHRIFT

(11) **EP 2 523 700 B1**
(45) Veröffentlichungstag und Bekanntmachung des Hinweises auf die Patenterteilung: **07.03.2018**
(21) Anmeldenummer: 11705422.1
(22) Anmeldetag: 12.01.2011
(51) Int. Cl.: A61L 24/00, A61L 24/04

(54) **ZUSAMMENSETZUNG ZUR ERZEUGUNG EINER TEMPORÄREN DARMOKKLUSION**
COMPOSITION FOR PRODUCING A TEMPORARY INTESTINAL OCCLUSION
COMPOSITION SERVANT À RÉALISER UNE OCCLUSION INTESTINALE TEMPORAIRE

(30) Priorität: 13.01.2010 AT 252010
(43) Veröffentlichungstag der Anmeldung: 21.11.2012
(73) Patentinhaber: Bischof, Georg, 1180 Wien (AT)
(72) Erfinder: Bischof, Georg, 1180 Wien (AT)
(74) Vertreter: Ellmeyer, Wolfgang
(86) Internationale Anmeldenummer: PCT/AT2011/000015
(87) Internationale Veröffentlichungsnummer: WO 2011/085424

(56) Entgegenhaltungen:
- EP-A1- 1 681 065
- WO-A1-97/22372
- WO-A2-2008/072102
- US-A1- 2001 036 451
- US-A1- 2002 176 893
- US-A1- 2006 222 596
- US-A1- 2006 269 512

## Beschreibung

Die vorliegende Erfindung betrifft das Gebiet der medizinischen Endoskopie und Endochirurgie, genauer gesagt jenes der Enteroskopie und -chirurgie.

Endoskopie ist ein seit vielen Jahrzehnten etabliertes Diagnoseverfahren in der Human- und Veterinärmedizin. Die hierfür verwendeten Endoskope haben sich kontinuierlich weiterentwickelt und ermöglichen heutzutage nicht nur einfaches Beleuchten und Abbilden des Körperinneren, z.B. unter Verwendung von Lichtleiterfasern, sondern sind auch für die Durchführung minimalinvasiver operativer Eingriffe ausgerüstet. So umfassen moderne Endoskope neben der Faseroptik beispielsweise Luftinsufflatoren oder Gaspumpen, Irrigatoren, Absaugpumpen sowie flexible Werkzeuge, wie z.B. Kanülen zur Injektion, Greif- oder Schneidwerkzeuge oder Drahtelektroden zur Koagulation mit elektrischem Strom. Zur Einführung der für den jeweiligen Eingriff erforderlichen Gerätschaften weist das Endoskop mehrere Kanäle auf.

Speziell bei enteroskopischen Eingriffen im Darm von Mensch und Tier treten, trotz vorhergehender Gabe von Abführmitteln, häufig Komplikationen aufgrund von die zu untersuchende und/oder operativ zu behandelnde Stelle passierendem Stuhl auf, was den Vorgang nicht nur erschwert und verzögert, sondern im Falle von operativen Eingriffen, z.B. zur Gewebeentnahme oder -entfernung, auch ein Infektionsrisiko für den Patienten darstellt. Somit wäre es wünschenswert, den Darm während eines endoskopischen Eingriffs temporär verschließen zu können.

In der Literatur hat der Erfinder hierzu lediglich die WO 2008/103891 ausfindig gemacht, deren Hauptanspruch zwar allgemein auf die Ausbildung eines Polymerpfropfens "an einer Stelle in einem Säugetier" abgestellt ist, indem eine viskose Polymerzusammensetzung bei Körpertemperatur verfestigen gelassen wird, deren einziger, im Rest der Anmeldung beschriebener Anwendungszweck jedoch der Verschluss von Arterien, d.h. Hämostase, ist. Grundlage der dortigen Offenbarung sind revers thermosensitive Polymere, d.h. Polymere, die bei Raumtemperatur wasserlöslich sind, bei Körpertemperatur jedoch aus der Lösung ausfallen. Beispiele sind Poloxamere, z.B. solche, die von BASF unter dem Handelsnamen Pluronics® vertrieben werden. US 2006/0222596 A offenbart die Verwendung von Biomaterialien zur Okklusion von Körperlumina wie z.B. des Darms. Hierzu wird ein Polyglycidylether direkt vor der Injektion mit einem Diamin als Vernetzer gemischt, was zur Bildung eines Hydrogels am Einsatzort führt.

Weitere Literaturstellen, die sich mit Hämostase unter Verwendung von natürlichen und künstlichen Polymeren, zum Teil unter zusätzlicher Gelierung der Polymerlösungen bei Temperaturänderung, beschäftigen, sind: G.M. Stiel et al., Z. Kardiol. 81(10), 543-545 (1992) (Kollagen als Polymer) und J. Raymond et al., Biomaterials 25, 3983 (2005) (Poloxamere); CN 1273860 (Poloxamer 407); KR 2002/023441 (N-Isopropylacrylamid-Copolymere); und US 5.894.022 (Albumin). Eine Verwendung dieser Materialien zum Verschließen des Darms ist in keinem der Zitate erwähnt oder nahe gelegt. Die beiden ersteren Literaturstellen sind auch in der WO 2008/103891 zitiert, wobei erwähnt wird, dass die Ausbildung eines das Blutgefäß verschließenden Pfropfens unter Einwirkung von Gelierung nachteilig sei, da ein solcher Pfropfen entweder nach seiner Auflösung die Gefahr eines erneuten Verschließens im Bereich engerer Gefäße mit sich bringt (betreffend das Kollagen bei Stiel et al.) oder sich nach einigen Minuten unerwünschterweise wieder auflöst (betreffend die Poloxamere bei Raymond et al.).

Für die Zwecke der vorliegenden Erfindung wären derartige Materialien, die durch eine Temperaturänderung zur Zustandsänderung bewegt werden, nur bedingt geeignet, da - im Gegensatz zur Verwendung in Blutgefäßen - im Lumen des vor dem Eingriff im Wesentlichen entleerten Darms keine ausreichend rasche Erwärmung der viskosen Polymerlösung erzielbar wäre, um einen Polymerpfropfen zu bilden, bevor größere Anteile der Lösung von der Okklusionsstelle abgeflossen sind.

Ziel der Erfindung war vor diesem Hintergrund die Bereitstellung eines geeigneten Materials zur Ausbildung einer vorübergehenden Okklusion des Darms.

### OFFENBARUNG DER ERFINDUNG

Die vorliegende Erfindung betrifft daher eine verfestigbare Zusammensetzung zur Verwendung zur Erzeugung einer vorübergehenden Okklusion des Darms eines Säugetiers, wobei die Zusammensetzung fließfähig und an einer gewünschten Stelle im Darm zu einem festen Pfropfen verfestigbar ist, dessen Struktur zur anschließenden, zumindest teilweisen Aufhebung der Okklusion veränderbar ist, sowie andererseits eine solche Zusammensetzung selbst. Eine solche erfindungsgemäße Zusammensetzung kann beispielsweise durch einen der Kanäle eines üblichen Endoskops an die gewünschte Stelle gepumpt und dort verfestigt werden, wobei weder die Art und Weise der Verfestigung noch die Art der Entfernung des Pfropfens nach Beendigung des Eingriffs speziell eingeschränkt sind, wie nachstehend näher ausgeführt wird. Wesentlich ist lediglich, dass der Pfropfen nach der Verfestigung ausreichende Festigkeit für einen ausreichend langen Zeitraum aufweist, um eine Barriere für zu dieser Stelle gelangenden Stuhl zu bilden, so dass der behandelnde Arzt stromab des Pfropfens den endoskopischen Eingriff ungehindert vornehmen kann.

Der Pfropfen braucht dafür den Darm nicht notwendigerweise zu 100 % zu verschließen, muss aber zumindest festen oder halbfesten Stuhl am Passieren der Einsatzstelle hindern können, womit der Pfropfen auch beispielsweise eine poröse Struktur aufweisen kann. Vorzugsweise wird jedoch auch eine Passage von dünnflüssigeren Exkrementen verhindert, was eine dichtere Konsistenz des Pfropfens bedingt. Da zudem die Weite des zu okkludierenden Darmlumens stark schwankt (z.B. Dünndarm ca. 2,5 cm, Dickdarm ca. 6 cm), kann die Zusammensetzung der Erfindung nach ihrer Verfestigung im Darm somit - je nach gewünschter Anforderung und je nach vorgesehener Einsatzstelle im Darm - eine große Bandbreite möglicher Beschaffenheiten aufweisen, die später ausführlicher diskutiert werden.

Die Zusammensetzung ist eine Lösung, Suspension oder Dispersion zumindest eines natürlichen oder künstlichen Polymers und ist vorzugsweise durch einen oder mehrere aus der aus Quellung, Koagulation, Polymerisation und Vernetzung bestehenden Gruppe ausgewählte Prozesse zu dem festen Pfropfen verfestigbar. Auf diese Weise ist einerseits der oben erwähnte Transport der Zusammensetzung zur gewünschten Einsatzstelle im Darm durch ein herkömmliches Endoskop und andererseits ihre dortige, möglichst rasche Verfestigung zu einem Pfropfen gewährleistet, der in der Lage ist, zu dieser Stelle gelangende Exkremente wirksam aufzuhalten. Erfindungsgemäß werden Polymere eingesetzt, deren Lösungen, Suspensionen oder Dispersionen thixotropes Verhalten zeigen, d.h. bei Energiezufuhr fließfähig sind, bei Wegfall der Energiequelle jedoch wieder zu einem festen Gel erstarren und so den Pfropfen bilden. Bekannte Beispiele hierfür sind entsprechende flüssige Formulierungen von Polyethylenglykolen (PEG), Polysacchariden wie Alginaten, Dextranen oder Celluloseethern, z.B. Carboxymethylcellulose-Derivaten, sowie manche der zuvor als gelbildende Polymere genannten Verbindungen.

Mit "Okklusionsstelle" ist hierin jene Stelle gemeint, an welcher der temporäre Darmverschluss vorgenommen werden soll. Aufgrund der Fließfähigkeit der erfindungsgemäßen Zusammensetzungen müssen diese mitunter an eine Position befördert werden, die - räumlich gesehen - etwas oberhalb der späteren Lage des verfestigten Pfropfens liegt, weswegen in der Definition der Okklusionsstelle ein Schwankungsbereich, z.B. von etwa 10 cm, enthalten ist. Zudem ist dem Fachmann klar, dass die Verfestigung in allen von der Erfindung umfassten Fällen möglichst rasch vonstatten gehen sollte, um ein Abfließen von der gewünschten Position weitestgehend zu verhindern. Daher sollten auch die allermeisten erfindungsgemäßen Zusammensetzungen eine solche Viskosität aufweisen, dass sie gerade noch fließfähig sind, um in den Darm pumpbar zu sein, sich aber vor Ort sehr rasch, vorzugsweise binnen Sekunden, verfestigen lassen. Ausnahmen sind beispielsweise viskoelastische oder thixotrope Zusammensetzungen, deren Viskosität im fließfähigen Zustand auch von der zugeführten Energiemenge abhängt.

Die Zusammensetzung muss, wie oben erwähnt, zu einem Pfropfen verfestigbar sein, dessen Struktur zur anschließenden, zumindest teilweisen Aufhebung der Okklusion veränderbar ist, wobei die Strukturänderung vorzugsweise eine zumindest teilweise Zerstörbarkeit auf mechanische, physikalische und/oder chemische Weise ist. Unter zumindest teilweiser Zerstörung der Struktur ist hierin im weitesten Sinne jegliche Strukturänderung des Pfropfens zu verstehen, die bewirkt, dass eine größere Menge an Exkrementen die Stelle passieren kann als unmittelbar nach der Ausbildung der Darmokklusion an dieser Stelle. Eine solche Strukturänderung kann von selbst erfolgen und/oder durch äußere Einwirkung herbeigeführt werden, wobei "von selbst" erfolgende Änderungen auch durch die im Darm herrschenden Bedingungen, wie z.B. den pH-Wert der Darmschleimhaut oder in den Darm ausgeschiedene Enzyme, ausgelöst werden können.

Unter "mechanische Zerstörung der Pfropfenstruktur" fallen jedwede vom behandelnden Arzt unter Verwendung geeigneter Werkzeuge vorgenommene Handlungen, durch die die Darmokklusion zumindest teilweise wieder aufgehoben wird und vorzugsweise die Strukturintegrität des Pfropfens beeinträchtigt oder zerstört wird. Für diesen Zweck können entweder die diversen Greif- oder Schneidewerkzeuge benutzt werden, mit denen herkömmliche Endoskope standardmäßig ausgerüstet oder ausrüstbar sind, wie eingangs bereits beschrieben wurde, oder es können spezielle Werkzeuge hierfür konzipiert werden. So kann der Pfropfen, je nach dessen Konsistenz, etwa mit einer Zange oder einem Skalpellen durchtrennt, mit einem Greifer zerrissen oder mit einem Dorn durchbohrt werden, um die Okklusion des Darms zumindest teilweise wieder aufzuheben.

Ebenfalls umfasst ist das Ergreifen des Pfropfens nach Beendigung des endoskopischen Eingriffs mit einem am Ende des Endoskops montierten Greifwerkzeug und anschließendes Herausziehen des - mitunter im Wesentlichen intakten - Pfropfens aus dem Darm über den natürlichen Darmausgang. Für diesen Zweck ist unter der obigen Anforderung einer veränderbaren Struktur des Pfropfens zur zumindest teilweisen aufhebung der Okklusion eine elastische Nachgiebigkeit bei gleichzeitiger ausreichender Konsistenz des Pfropfenmaterials zu verstehen, um den Verschlusspfropfen von der Darmschleimhaut ablösen und durch den Darm transportieren zu können, ohne die Mukosa zu verletzen. Auch umfasst sind Fälle, in den der Pfropfen mit einem Werkzeug ergriffen und von der Darmwand abgelöst wird, wonach der Transport zum After jedoch nicht manuell, sondern durch den Druck nachkommender Exkremente auf den Pfropfen erfolgt. Aufgrund des Risikos eines erneuten, unerwünschten Darmverschlusses auf dem Weg zum Darmausgang ist diese Ausführungsform freilich nicht bevorzugt.

Ebenfalls möglich ist zunächst eine mechanische Zerstörung des Pfropfens, gefolgt von einer Auflösung desselben, wobei die Auflösung wiederum von selbst erfolgen oder vom behandelnden Arzt initiiert werden kann und mehr oder weniger rasch erfolgen kann. Ein bevorzugtes Beispiel besteht darin, einen aufgrund von viskoelastischem bzw. thixotropem Verhalten gebildeten Pfropfen durch Zufuhr von mechanischer Energie in Form von Scherspannung, d.h. durch einfaches Eindringen in den Pfropfen und Durchführung einer Rührbewegung, gegebenenfalls unter Ausübung von leichtem Druck auf die Darmwand, wieder in einen fließfähigen Zustand überzuführen, wodurch das Pfropfenmaterial zumindest teilweise von der Okklusionsstelle abfließt, so die Okklusion aufhebt und in der Folge durch das Darmsekret allmählich aufgelöst und entweder über den natürlichen Darmausgang entfernt oder über die Darmschleimhaut resorbiert wird. Zumindest teilweise Resorption stellt in allen Fällen der Pfropfenentfernung eine erfindungsgemäß bevorzugte Variante dar.

Unter physikalische Strukturänderungen des Pfropfens fallen alle Vorgänge, bei denen die Struktur ohne Zuhilfenahme von Werkzeugen ausreichend verändert wird, um die Darmokklusion zumindest teilweise wieder aufzuheben, ohne dass es zu substanziellen chemischen Veränderungen im Pfropfenmaterial kommt. Dazu zählen beispielsweise Strukturveränderungen aufgrund von Temperaturerhöhung oder -senkung. Für die Zwecke hierin werden unter physikalischen Strukturänderungen in weiterem Sinne der Definition jedoch auch Quellung bzw. Schrumpfung, womit der entgegengesetzte Vorgang von Quellung gemeint ist, verstanden, wiewohl es bei diesen Prozessen zwangsläufig zu deutlichen Änderungen chemischer Bindungszustände im Pfropfenmaterial kommt.

Vom behandelnden Arzt aktiv bewirkte Temperaturänderungen können beispielsweise durch Bestrahlung des Pfropfens mit Infrarotlicht herbeigeführt werden, wofür erneut die in modernen Endoskopen vorgesehenen Lichtleiter unter Verwendung geeigneter Lichtquellen zum Einsatz kommen können, obwohl bessere Ergebnisse unter Verwendung spezieller, an den Infrarotbereich angepasster Lichtleitkabel erzielbar sind.

Wiewohl spontane Quellung/Schrumpfung aufgrund der im Darm herrschenden Bedingungen, z.B. unter Beteiligung von den Pfropfen erreichenden Exkrementen, nicht ausgeschlossen ist, erfordern diese Vorgänge in der Regel die gezielte Zufuhr eines Reaktionspartners, nämlich eines Quellmittels, wie z.B. Wasser oder Lösungsmittel, bzw. eines Schrumpfungsmittels oder "Anti-Quellmittels", also eines Mittels, das bewirkt, dass ein gequollenes Polymer (z.B. ein Hydrogel) seinen Quellgrad und somit auch sein Volumen verringert. Als Schrumpfungsmittel können beispielsweise Salzlösungen oder andere Lösungen oder Suspensionen ionischer Gelöststoffe dienen, die in das gequollene Polymer eindringen und dessen Nebenvalenzen absättigen, so dass diese kein Wasser oder Lösungsmittel mehr zu binden vermögen, wodurch der Quellgrad gesenkt und eine Schrumpfung des Pfropfens ausgelöst wird.

Vorzugsweise ist die Struktur des Pfropfens durch Quellung bzw. - falls bereits die Verfestigung der erfindungsgemäßen Zusammensetzung zur Bildung des Pfropfens aufgrund von Quellung erfolgt ist - weitere Quellung zumindest teilweise zerstörbar, so dass die Zufuhr eines Quellmittels bzw. von weiterem Quellmittel ausreicht, um die gewünschte Strukturänderung zu bewirken. Das weitere Quellmittel kann dabei dasselbe wie das für den ersten Quellvorgang verwendete oder auch ein anderes Mittel sein. Beispielsweise kann die erste Quellung zur Verfestigung der erfindungsgemäßen Zusammensetzung unter Verwendung von Wasser als Quellmittel erfolgen, und die Struktur des so gebildeten Pfropfens durch Einsatz eines wässrigen Lösungsmittelgemischs als zweites Quellmittel wieder (zumindest teilweise) zerstört werden, oder umgekehrt.

Die Strukturänderung durch Quellung kann dabei sowohl ein - mehr oder weniger vollständiges - In-Lösung-Gehen des Pfropfenmaterials umfassen als auch ein bloßes Erweichen desselben, so dass der (erneut) gequollene, weiche Pfropfen beispielsweise leicht(er) mechanisch zerstörbar wird als zuvor, oder aber so weich und nachgiebig wird, dass er durch den Druck von ankommendem Stuhl auf natürliche Weise aus dem Darm befördert werden kann, ohne dass das Risiko eines erneuten Darmverschlusses auf dem Weg zum After besteht. Letztere Variante stellt daher ein Beispiel für eine Strukturänderung durch eine Kombination aus physikalischer bzw. physikalisch-chemischer Zerstörung und mechanischer Entfernung dar.

Alternativ oder zusätzlich zur oben beschriebenen Quellung kann die Struktur des Pfropfens auch durch Bestrahlung mit elektromagnetischen Strahlen zumindest teilweise zerstört werden. Beispielsweise kann die erfindungsgemäße Zusammensetzung ein Polymer umfassen, das einen Photosäurebildner enthält, der bei Bestrahlung mit Licht geeigneter Wellenlänge eine Säure freisetzt, die die gewünschte Strukturänderung des Pfropfens bewirkt. Das Polymer kann aber beispielsweise auch einen Photoinitiator enthalten, der bei Bestrahlung eine Polymerisation oder Vernetzung innerhalb des Pfropfenmaterials auslöst, und so dessen Strukturänderung herbeiführen. Letztere kann beispielsweise darauf beruhen, dass durch derartige Photopolymerisation des Pfropfens ein porös(er)es und damit durchlässigeres Polymernetzwerk oder aber auch ein Material mit höherer Quellbarkeit erzeugt wird. Letzterer Fall ist ein Beispiel für eine Strukturänderung aufgrund einer Kombination aus Bestrahlung und Quellung.

Alternativ oder zusätzlich zu Quellung und Bestrahlung kann die Struktur des Pfropfens durch Spaltung chemischer Bindungen zumindest teilweise zerstörbar sein, wobei hierin unter spaltbaren chemischen Bindungen - zur gedanklichen Abgrenzung gegenüber der obigen Definition physikalischer Strukturänderungen - vorwiegend kovalente Bindungen zu verstehen sind, da es bei Vorgängen wie Quellung oder Schrumpfung, aber auch Koagulation, zu einer "Spaltung" von Nebenvalenzbindungen wie etwa von koordinativen Bindungen, z.B. Wasserstoffbrückenbindungen, oder von lonenbindungen kommt.

Zum Zwecke der Spaltung solcher chemischer Bindungen umfasst die erfindungsgemäße Zusammensetzung vorzugsweise ein oder mehrere Polymere und/oder ein oder mehrere bei Verfestigung durch Polymerisation bzw. Vernetzung in ein Polymernetzwerk einzupolymerisierende Monomere, die jeweils labile Bindungen enthalten. Unter "labiler Bindung" ist hierin im weitesten Sinne eine Bindung zu verstehen, die auf relativ einfache Weise und vorzugsweise unter schonenden Bedingungen chemisch spaltbar sind. Vorzugsweise sind die labilen Bindungen aus hydrolyse-, licht- und temperaturempfindlichen Bindungen ausgewählt, und zwar bevorzugt aus der aus Acetal-, Ketal-, Ester-, Orthoester-, Azo-, Ether- und Anhydridbindungen bestehenden Gruppe. Allerdings sind auch enzymatisch spaltbare Bindungen bevorzugt, wobei besonders bevorzugt Bindungen gewählt werden, die durch körpereigene, in den Darm sekretierte Verdauungsenzyme gespalten werden können, wobei die Art der Bindung erneut von der beabsichtigten Position des Eingriffs und damit der Okklusion abhängt.

Beispielsweise kann unter Verwendung eines oben erwähnten Photosäurebildners durch Bestrahlung eine Säure gebildet werden, die zur Spaltung von säurelabilen Bindungen im Pfropfen führt, was somit ein Beispiel für eine Strukturänderung durch eine Kombination aus Bestrahlung und Spaltung chemischer Bindungen darstellt. Aus Gründen der physiologischen Unbedenklichkeit sollte eine entweder aus einem Photosäurebildner unter Bestrahlung freigesetzte oder eine gezielt, z.B. als Reagens(lösung), zugesetzte Säure keine allzu starke Säure sein, weswegen die labilen (in diesem Fall: säurelabilen) Bindungen bereits in schwach saurem Milieu spaltbar sein sollten. Analoges gilt bei Verwendung von Basen für basenlabile Bindungen, die ebenso Beispiele für hydrolyseempfindliche Bindungen darstellen.

Besonders bevorzugt sind daher Verbindungen mit säure- oder basenlabilen Bindungen, die auch in Gegenwart schwacher Säuren oder Basen spaltbar sind, wozu beispielsweise Carbonate, Acetale, Anhydride und Orthoester zählen. Dadurch ist ein Pfropfenmaterial erhältlich, das auch bei dem - je nach Zusammensetzung der vom Säugetier metabolisierten Nahrung und Lage der Operationsstelle im Darm - schwach sauren oder schwach basischen pH-Wert des Speisebreis bzw. der Exkremente durch diese spaltbar ist, so dass sich der Pfropfen auch ohne äußere Einwirkung, zumindest teilweise, allmählich auflöst. Beispielsweise schwankt der pH-Wert im Zwölffingerdarm etwa zwischen 5 und 8, im Dickdarm zwischen etwa 5,5 und 6,8.

In der Praxis kann der pH-Wert des Darms an der geplanten Okklusionsstelle in gewissem Ausmaß dadurch gesteuert werden, dass dem Patienten über einen bestimmten Zeitraum vor dem Termin des Eingriffs eine speziell für diesen Zweck abgestimmte Kost verabreicht wird.

Ebenfalls bevorzugt sind Verbindungen mit licht- oder temperaturempfindlichen Bindungen, wie z.B. Azo- und Diazo-Gruppen, um die Struktur des aus der erfindungsgemäßen Zusammensetzung gebildeten Pfropfens photolytisch bzw. thermolytisch verändern oder zerstören zu können. In beiden Fällen kann der Auslöser erneut eine Bestrahlung über den Lichtleiter des Endoskops sein.

Ein großer Vorteil der erfindungsgemäßen Zusammensetzung besteht darin, dass sie über den natürlichen Darmausgang an die gewünschte Stelle im Darm zuführbar ist, z.B. indem sie durch einen Kanal eines herkömmlichen Endoskops an die Einsatzstelle gepumpt und dort auf geeignete Weise zum Pfropfen verfestigt wird. Es sind somit keinerlei Traumata der Darmwand vonnöten, um die Darmokklusion unter Verwendung der erfindungsgemäßen Zusammensetzung herbeizuführen. Der Darmverschluss kann somit auf nichtinvasive bzw. minimalinvasive (je nach Definition) und auf für den behandelten Patienten schonendste Weise erzeugt werden.

Auch die Entfernung des aus der Zusammensetzung gebildeten festen Pfropfens erfolgt vorzugsweise, gegebenenfalls nach vorheriger, zumindest teilweiser Zerstörung seiner Struktur, über den natürlichen Darmausgang. Wenn die zumindest teilweise Zerstörung der Pfropfenstruktur einen chemischen oder biologischen Abbau umfasst, kann jedoch - alternativ oder zusätzlich zur Entfernung über den natürlichen Darmausgang - auch eine zumindest teilweise Resorption durch die Darmwand des Patienten erfolgen.

Abgesehen von den zur Pfropfenbildung unabdinglichen Bestandteilen kann die erfindungsgemäße Zusammensetzung beliebige weitere Komponenten enthalten, solange dieser eine Anwendung im Darm von Säugetieren nicht entgegenstehen und die Wirkung der Erfindung nicht beeinträchtigen. Explizit seien hier Viskositätsmodifikatoren, wie z.B. Verdicker oder Fließhilfen, Lösungsvermittler oder Löslichkeitsverbesserer, wie z.B. Tenside und Emulgatoren, Geliermittel, Schaumstabilisatoren und Haftmittel genannt. Letztere sollen insbesondere eine gute Haftung des Pfropfens an der Darmwand fördern, wofür beispielsweise stark klebrige natürliche Polymere, wie z.B. die bereits erwähnten Tragant- und Karaya-Gummen, in Frage kommen. Diese können in der Zusammensetzung entweder lediglich eingemischt enthalten sein, z.B. wenn die Pfropfenbildung ausschließlich aufgrund von Quellung erfolgt, oder können so modifiziert sein, dass sie an etwaigen Polymerisationsreaktionen teilnehmen. Dabei können derartige Derivate sowohl die einzigen polymerisierbaren Verbindungen darstellen und zu den Pfropfen bildenden Homopolymeren polymerisiert werden, oder sie können mit anderen polymerisierbaren Bestandteilen der Zusammensetzung Copolymere bilden.

Die Patienten, bei denen die erfindungsgemäßen Zusammensetzung Verwendung finden soll, sind zwar nicht speziell eingeschränkt und können beispielsweise Haus- oder Nutztiere sein. Nicht zuletzt aufgrund der nicht unbeträchtlichen Behandlungskosten für endoskopische Eingriffe wird es sich jedoch im Normalfall um menschliche Patienten handeln.

### KURZBESCHREIBUNG DER ZEICHNUNGEN

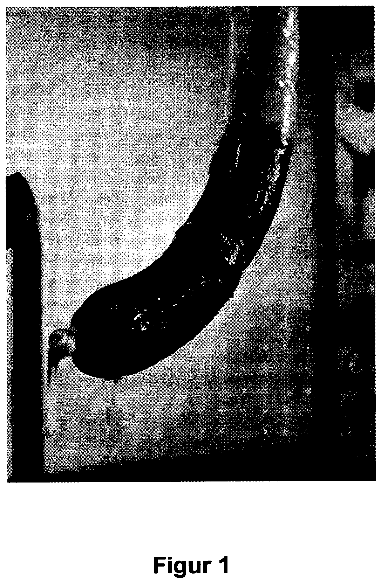

Die Fig. 1 bis 3 sind Fotografien eines Modellversuchs der Ausführung der Erfindung in einem Schweine-Naturdarm.

Nachstehend wird die vorliegende Erfindung anhand von nichteinschränkenden Fallbeispielen und konkreten Modellversuchen mit Schweinedarm-Abschnitten unter Bezugnahme auf die Figuren näher beschrieben.

### BEISPIELE

Da sowohl die Komponenten als auch die Eigenschaften der erfindungsgemäßen Zusammensetzungen maßgeblich von der Art und Weise der Bildung des die Okklusion bewirkenden Polymerpfropfens sowie von der Art der Entfernung desselben bestimmt werden, werden die folgenden Beispiele nach diesen beiden Verwendungsaspekten zu Gruppen zusammengefasst. So beschreiben die Beispiele 1 bis 13 unterschiedliche Arten der Verfestigung zur Pfropfenbildung, während sich die anschließenden Beispiele 14 bis 28 die Aufhebung der Okklusion illustrieren, wobei in der Praxis beliebige Kombinationen aus Pfropfenbildung und -entfernung möglich sind, auch wenn dies in den nachstehenden Beispielen nicht für jeden Fall explizit angegeben ist. Die Zufuhr der erfindungsgemäßen Zusammensetzungen an die Okklusionsstelle erfolgt in jedem der Beispiele in einfacher Weise über die Kanäle eines Endoskops, wiewohl - zumindest in der Theorie - auch andere Optionen existieren, die jeweilige Zusammensetzung an die Okklusionsstelle zu transportieren, z.B. über gesonderte Schläuche oder Kanäle oder, z.B. mittels einer Spritze, durch die Darmwand hindurch. Aufgrund ihrer daraus resultierenden Komplexheit werden derartige Operationen in der Praxis freilich kaum zum Einsatz kommen.

Wie bereits zuvor erwähnt wurde, können erfindungsgemäße Zusammensetzungen beliebige zusätzliche Bestandteile enthalten, wobei der einschlägige Fachmann auf dem Gebiet der medizinischen Chemie problemlos in der Lage ist, geeignete Formulierungen zu konzipieren, deren Bestandteile sowohl untereinander als auch für die damit in Kontakt kommende Darmschleimhaut verträglich sind.

### A) Unterschiedliche Arten der Ausbildung der Darmokklusion

### A1. Verfestigung der Zusammensetzung durch Gelierung/Koagulation

### Beispiele 1 und 2 - Zweikomponenten-Systeme

Eine wässrige kolloidale Lösung oder Suspension eines quellfähigen Polymers mit einer Konzentration in der Nähe des Gelierpunkts wird als Formulierung A durch einen Kanal eines herkömmlichen Endoskops an die gewünschte Okklusionsstelle gepumpt. Durch denselben oder einen zweiten Kanal des Endoskops wird eine ausreichende Menge einer fließfähigen Formulierung B eines Geliermittels an dieselbe Stelle im Darm abgegeben. Die Viskositäten und der Pumpendruck werden jeweils so gewählt, dass einerseits rascher Transport der beiden Formulierungen an die Zielstelle, um beispielsweise starke Temperaturänderungen der jeweiligen Formulierungen im Kanal zu verhindern, und andererseits eine rasche Vermischung derselben gewährleistet sind, ohne dass nennenswerte Mengen der beiden Flüssigkeiten von der Okklusionsstelle abfließen.

### Beispiel 1 (nicht erfindungsgemäß): Das Polymer selbst dient als Geliermittel

Die Formulierungen A und B sind kolloidale Lösungen von Agar-Agar, Gelatine, Carrageen o.dgl., die gegebenenfalls chemisch modifiziert wurden, wobei die optionale Modifikation beispielsweise zur Verschiebung der nachstehend als "Geltemperatur" bezeichneten Temperatur des Übergangs zwischen Gel- und Sol-Zustand, der z.B. bei Gelatine normalerweise bei etwa 35 °C und bei Agar-Agar bei etwa 45 °C erfolgt, und zur Erhöhung der Löslichkeit dienen kann, da ansonsten bereits aus 1%iger Lösung feste Gele gebildet werden. Alternativ oder zusätzlich dazu kann ein Tensid, z.B. ein nichtionogenes Tensid wie etwa ein Ethylenoxid-Propylenoxid-Copolymer, oder ein anderes geeignetes Mittel als Löslichkeitsverbesserer zugesetzt werden, das bewirkt, dass die Gelierung erst bei höheren Konzentrationen erfolgt.

Ohne Zusatz eines solchen Löslichkeitsverbesserers kann Formulierung A eine wässrige Lösung mit einer Konzentration knapp unter der Geliergrenze, z.B. eine maximal ca. 0,9%ige Lösung, sein, Formulierung B hingegen eine über die Geltemperatur erwärmte wässrige Lösung mit einer höheren Konzentration, die vom Mengen- und Mischungsverhältnis der beiden Komponenten abhängt. Beispielsweise werden 100 ml einer 0,9%igen Formulierung A und 10 ml einer warmen 15%igen Formulierung B gleichzeitig zur Okklusionsstelle zugeführt. Unter "warm" ist hier eine Temperatur zu verstehen, die ausreicht, dass die Lösung trotz allfälliger Abkühlung im Endoskopkanal beim Austritt aus Letzterem in das Darmlumen eine Temperatur oberhalb der Geltemperatur aufweist, um Gelierung im Kanal zu verhindern. Je nach Fließgeschwindigkeit, Länge des Fließwegs und Ausmaß des Wärmeübergangs zwischen transportierter Lösung und dem Kanalmaterial sollte eine Temperatur von 50 bis 60 °C bei Eintritt in den Kanal ausreichen. Vorzugsweise liegt die Temperatur bei Austritt nur geringfügig, z.B. < 5 °C, oberhalb der Geltemperatur, damit eine ausreichend rasche Abkühlung erfolgt, um den Pfropfen zu bilden, ohne dass übermäßige Mengen der Lösung abfließen.

Die beiden Lösungen ergeben ein Gemisch mit einer Konzentration oberhalb der Geliergrenze und einer Temperatur unterhalb Geltemperatur, so dass das Polymer sofort einen festen Gelpfropfen mit einem Durchmesser von rund 4 bis 5 cm bildet, der den Darm an dieser Stelle verschließt.

Alternativ dazu kann als Formulierung B auch einfach Wasser oder physiologische Kochsalzlösung verwendet werden. Die Gelierung wird in diesem Fall dadurch initiiert, dass als Formulierung A eine Lösung des Polymers mit einer Konzentration deutlich oberhalb der Geliergrenze, z.B. eine 5%ige Lösung, und einer Temperatur oberhalb der Geltemperatur an die Okklusionsstelle gepumpt wird und als Formulierung B beispielsweise Wasser mit Raum- oder einer darunter liegenden Temperatur, z.B. 10 bis 15 °C, zugeführt wird, um so rasch ein Gemisch mit einer Temperatur unterhalb der Geltemperatur zu bilden.

### Beispiel 2 (nicht erfindungsgemäß): Zusätzliches Geliermittel Formulierung A ist eine in Beispiel 1 beschriebene etwa 0,9%ige Lösung von Gelatine, Agar-Agar oder eines ähnlichen, gegebenenfalls modifizierten Polypeptids oder Polysaccharids, und als Formulierung B dient eine Lösung eines separaten Geliermittels, wie z.B. gesättigte Kochsalzlösung. Beim Vermischen der beiden Komponenten wird die Löslichkeit des Polymers durch das Salz verringert, so dass das Polymer koaguliert und zum gewünschten Pfropfen geliert.

### Beispiele 3 bis 6 - Einkomponenten-Systeme

Unter Ausnutzung ähnlicher Mechanismen wie oben für Zweikomponenten-Systeme beschrieben und/oder rheologischer Effekte kann die Erfindung auch in Form einer einzigen fließfähigen Zusammensetzung in die Praxis umgesetzt werden.

### Beispiel 3 (nicht erfindungsgemäß): Gelierung durch spontane Temperaturänderung

Eine Lösung eines der oben erwähnten Polypeptide oder Polysaccharide mit einer Konzentration oberhalb der Geliergrenze wird in erwärmtem Zustand in den Darm eingeleitet, d.h. wiederum mit einer Temperatur, die ausreicht, damit die Lösung bei Erreichen der Okklusionsstelle eine Temperatur knapp oberhalb der Geltemperatur aufweist. Bei Abkühlung geliert die Zusammensetzung und bildet die Okklusion. Alternativ dazu können jedoch auch Lösungen oder Suspensionen von Polymeren eingesetzt werden, deren Sol/Gel-Übergang in umgekehrter Richtung, d.h. Gelierung unter Erwärmung, erfolgt. Beispiele hierfür sind unter anderem auch die nach dem Stand der Technik bekannten und bereits einleitend erwähnten Poloxamere, die als kalte Lösungen, z.B. mit einer Temperatur von etwa 15 °C, zugeführt werden und bei Erwärmung auf die jeweilige Geltemperatur, z.B. 20 °C, gelieren können. Allerdings ist zu beachten, dass eine Gelbildung unter Erwärmung üblicherweise deutlich langsamer erfolgt als jene bei Abkühlung, so dass diese Variante erfindungsgemäß nicht bevorzugt ist.

### Beispiel 4 (nicht erfindungsgemäß): Gelierung durch gezielte Erwärmung

Eine Lösung der eben beschriebenen Problematik von bei Temperaturerhöhung gelierenden Polymeren besteht in zusätzlicher Erwärmung der Lösungen vor Ort. So kann wie in Beispiel 3 erwähnt eine etwa 15 °C kalte Lösung an die Okklusionsstelle gepumpt und über das Lichtleiterkabel des Endoskops sofort mit Infrarotlicht bestrahlt werden, um so eine ausreichend rasche Erwärmung und Gelierung zu bewirken. Dieses Beispiel illustriert somit eine Pfropfenbildung durch eine Kombination aus Gelierung und Bestrahlung.

### Beispiel 5 (nicht erfindungsgemäß): Gelierung durch Wasserentzug

Eine wie in Beispiel 1 beschriebene, etwa 0,9%ige Gelatine- oder ähnliche Lösung, die gegebenenfalls durch Zusatz eines Verdickers wie etwa eines Celluloseethers, z.B. Hydroxyethylcellulose, oder von Polyvinylalkohol auf eine solche Viskosität eingestellt wurde, dass sie bei Körpertemperatur gerade noch fließfähig ist, wird an die Okklusionsstelle gepumpt. Vorzugsweise wird die Zusammensetzung dabei vor dem Einleiten in den Endoskopkanal auf Körpertemperatur vorgewärmt. Durch im Darm erfolgende Resorption von Wasser aus der Zusammensetzung erhöht sich die Viskosität der Zusammensetzung weiter, bis deren Konzentration schließlich die Gelgrenze überschreitet und zum Pfropfen geliert.

### Beispiel 6: Gelierung aufgrund von Viskoelastizität oder Thixotropie

Hier kommt eine kolloidale Lösung, Suspension oder Dispersion eines körperverträglichen, natürlichen oder künstlichen Polymers zum Einsatz, die viskoelastisches oder thixotropes Verhalten zeigt. Die Formulierung enthält vorzugsweise ein oder mehrere geeignete Polymere, wie etwa entsprechende Polyethylenglykole, Polysaccharide wie Alginate, Dextrane oder Celluloseether, z.B. Carboxymethylcellulose-Derivate, in einer solchen Konzentration, dass die Formulierung ohne äußere Energiezufuhr nicht fließfähig ist, unter geringer Energiezufuhr aber durch den Kanal des Endoskops gepumpt werden kann. Zusätzlich oder alternativ dazu kann die Formulierung ein Menge eines (weiteren) physiologisch unbedenklichen Thixotropiermittels enthalten.

In der Praxis wird eine als Gel vorliegende Lösung, Suspension oder Dispersion eines entsprechenden Polymers durch Rühren verflüssigt und mit einem solchen Druck und zugehöriger Fließgeschwindigkeit durch einen Endoskop-Kanal an die Okklusionsstelle gepumpt, dass die resultierende Schubspannung ausreichend Energie bereitstellt, damit die Fließfähigkeit der Formulierung erhalten bleibt. Nach Austritt aus dem Kanal und entsprechendem Wegfall der Energiezufuhr erstarrt die Formulierung rasch wieder zu einem das Darmlumen verschließenden festen Gel.

### A2. Verfestigung der Zusammensetzung durch Polymerisation

### Beispiele 7 bis 10 - Einkomponenten-Systeme

In diesen Fällen wird eine polymerisierbare Zusammensetzung der Erfindung als Formulierung an die Okklusionsstelle gepumpt und durch Polymerisation zu einem festen Pfropfen verfestigt. Die Polymerisation erfolgt zwar in allen Fällen im Wesentlichen erst vor Ort, d.h. an der Okklusionsstelle, kann aber mitunter auch schon während des Transports der Zusammensetzung durch den Kanal einsetzen, solange die Fließfähigkeit gewahrt bleibt. Zudem sei allgemein für sämtliche Polymerisationsbeispiele - unabhängig davon, ob es sich um Ein- oder Zweikomponenten-Systeme handelt - erneut festgestellt, dass das jeweils gebildete Polymerisat entweder mit gegebenenfalls vorhandenem Lösungsmittel oder mit Wasser aus Darmsekreten oder beiden quellbar sein sollte.

### Beispiel 7 (nicht erfindungsgemäß): Polymerisation bei Raumtemperatur

Eine polymerisierbare Formulierung wird durch Vermischen eines geeigneten Mono- oder Prepolymers mit einem bereits bei Raumtemperatur reagierenden Initiator und gegebenenfalls einem Lösungsmittel, vorzugsweise Wasser, gebildet. Als Initiatoren eignen sich hierfür vor allem Redoxinitiatoren von radikalischer Polymerisation, wie z.B. die Systeme Fe²⁺/H₂O₂, Peroxysulfat/Metabisulfat, Peroxid/Thiosulfat oder Peroxysulfat/Thiosulfat, die allesamt wasserlöslich sind. Für Pfropfcopolymerisationen verschiedener Polysaccharid-Derivate, wie z.B. modifizierter Cellulose, reicht auch beispielsweise der Einsatz eines Cer(IV)-Salzes, da der Mehrfachzucker selbst als reduzierender Redoxpartner dient. Auch anionische oder kationische Initiatoren sind möglich, werden jedoch aufgrund des langsamen Reaktionsverlaufs ionischer Polymerisationen nicht bevorzugt.

Die polymerisierbaren Monomere und Prepolymere sind nicht eingeschränkt. Vorzugsweise werden wasserlösliche, zu mit Wasser quellbaren Polymeren reagierende, physiologisch unbedenkliche Verbindungen ausgewählt, noch bevorzugter solche mit ethylenischen Doppelbindungen, insbesondere solche, deren Polymerisate im Darm zu Verbindungen abbaubar sind, die als Nährstoffe resorbiert werden können, wie z.B. Vinylester von Fettsäuren, Aminosäuren oder Fruchtsäuren, z.B. von Milchsäure, Citronensäure oder Weinsäure. Diese ergeben bei der Polymerisation Derivate von Polyvinylalkohol, die durch Säure- oder Baseneinwirkung und/oder Esterasen zu Polyvinylalkohol (PVAL) und der entsprechenden Säure spaltbar sind. Bei Verwendung von Asparaginsäure bzw. von Citronen- oder Weinsäure oder ähnlichen Polycarbonsäuren können deren Di- bzw. Trivinylester - alleine oder zusätzlich zu Monovinylestern - eingesetzt werden, die als Vernetzer dienen. Dabei ist über das Verhältnis zwischen Mono- und Di- bzw. Triester-Monomeren der Vernetzungsgrad und damit auch die Quellbarkeit des Polymerisats steuerbar. Da starkes Quellen aus zuvor erwähnten Gründen in der Regel erwünscht ist, beträgt der Vernetzeranteil vorzugsweise nicht mehr als etwa 10 Mol-%, noch bevorzugter nicht mehr als 5 Mol-%.

Bei Verwendung von Prepolymeren anstelle von oder zusätzlich zu Monomeren ist ebenfalls auf Verträglichkeit und Abbaubarkeit zu achten. Bevorzugte Beispiele sind modifizierte Vertreter natürlicher Polymere, wie z.B. von Polypeptiden, Polysacchariden und dergleichen, wie bereits zuvor erwähnt wurde, insbesondere z.B. Vinyloxycarbonyloxy-Derivate von Gelatine, Hyaluronsäure oder Glykogen, d.h. Kohlensäuremonovinylester - z.B. solche von freien OH-, NH- oder NH₂-Gruppen im Prepolymer - die durch Decarboxylierung leicht zu PVAL und das entsprechende natürliche Polymer spaltbar sind.

In jedem Fall setzt die Polymerisation des bei Raumtemperatur wirksamen Initiators bereits beim Vermischen ein, kann jedoch z.B. durch Kühlung der Formulierung vor dem Einpumpen in den Endoskopkanal oder Zusatz geringer Mengen an Stabilisatoren in geringerem Ausmaß unterdrückt werden. Dennoch muss die Zufuhr zur Okklusionsstelle möglichst rasch, d.h. mit relativ hohem Druck und damit hoher Fließgeschwindigkeit erfolgen, um den Kanal nicht zu verstopfen. Durch die Erwärmung der Zusammensetzung im Körper beschleunigt sich die Polymerisationsreaktion und läuft rasch bis zur Vollständigkeit ab. Das Polymerisationsprodukt quillt in der Folge mit Wasser zum die Okklusion bildenden Pfropfen.

### Beispiel 8 (nicht erfindungsgemäß): Thermisch initiierte Polymerisation

Eine ähnliche Zusammensetzung wie in Beispiel 7 beschrieben, d.h. z.B. unter Verwendung von Vinylester- oder Vinylcarbonat-Derivaten als Monomere oder Prepolymere, allerdings mit einem thermischen Polymerisationsinitiator, wie z.B. einem organischen Peroxid, z.B. Dibenzoylperoxid oder Di-tert-butylperoxid, oder einer Azo-Verbindung. An der Okklusionsstelle erfolgt sofort eine Bestrahlung mit Infrarot-Licht über einen Lichtleiter in einem zweiten Kanal des Endoskops, am besten gleichzeitig mit dem Austreten der Formulierung aus dem ersten Kanal, um die Polymerisation in Gang zu setzen, bevor nennenswerte Mengen der Zusammensetzung abgeflossen sind. Bei der Bestrahlung ist freilich sorgfältig darauf zu achten, dass die Darmschleimhaut von der Bestrahlung ausgespart wird, um keine Verbrennungen zu verursachen.

### Beispiel 9 (nicht erfindungsgemäß): Photopolymerisation

Eine ähnliche Zusammensetzung wie in den beiden vorhergehenden Beispielen beschrieben wird mit einem Photoinitiator, gegebenenfalls unter zusätzlicher Verwendung eines Sensibilisators oder Co-Initiators, hergestellt. An der Okklusionsstelle wird die Formulierung erneut sofort bestrahlt, in diesem Fall mit Licht der geeigneten Wellenlänge im UV/VIS-Bereich, wo die gängigen Photoinitiatoren Absorption zeigen. Dabei ist vom behandelnden Arzt wiederum darauf zu achten, dass die umliegende Darmschleimhaut von der Bestrahlung möglichst verschont bleibt, um keine Irritationen auszulösen.

Illustrativ wird ein einfaches Hydroxyalkylphenon, wie z.B. 2-Hydroxy-2-methyl-1-phenyl-1-propanon (erhältlich als Darocur® 1173 von Ciba), das sehr rasche Reaktionsraten ermöglicht, als Initiator eingesetzt und mit Licht zwischen 200 und 340 nm bestrahlt. Die genaue Wellenlänge hängt von der Konzentration des Initiators ab, die vorzugsweise zwischen 0,001 % und 0,1 % beträgt, und zwar vorzugsweise in einem Gemisch aus Wasser und organischem Lösungsmittel wie etwa Wasser/Alkohol oder Wasser/Glykol(ether). Binnen wenigen Sekunden reagiert der überwiegende Teil der polymerisierbaren Gruppen, und es bildet sich ein gequollenes Polymerisat, das den gewünschten Pfropfen bildet.

### Beispiel 10 (nicht erfindungsgemäß): Polymerisation unter Schäumung

Eine Zusammensetzung wie in einem der Beispiele 7 bis 9 beschrieben, die jedoch zusätzlich ein Blähmittel enthält, wird an der Okklusionsstelle polymerisiert, wobei das Blähmittel bewirkt, dass das gebildete Polymer aufschäumt und so das Darmlumen vollständig verschließt. Als Blähmittel kann z.B. bei Verwendung von Azo-Verbindungen als Initiatoren der Initiator selbst dienen, und/oder ein oder mehrere Monomere oder Prepolymere enthalten funktionelle Gruppen, die während der Polymerisation ein Gas freisetzen, z.B. Carbonate oder Polyurethane, die unter Decarboxylierung, z.B. bei Wärmeeinwirkung, CO₂ abgeben, wodurch das Polymerisat zum Schaum aufgebläht wird. Zusätzlich kann die Zusammensetzung Schaumstabilisatoren enthalten, um die Festigkeit und Elastizität des Polymerschaums zu erhöhen. Beispiele sind etwa Fettsäurealkanolamide oder ethoxylierte Polysiloxane.

### Beispiele 11 bis 13 - Zweikomponenten-Svsteme

Auch durch Polymerisation härtbare erfindungsgemäße Zusammensetzungen können als Zweikomponenten-Systeme zweier Formulierungen A und B vorliegen, wobei die zweite Komponente vorzugsweise den Polymerisationsinitiator enthält, aber nicht (oder nicht nur) enthalten muss. Anstelle oder zusätzlich zum Initiator kann die zweite Komponente beispielsweise auch Quellmittel, ein oder mehrere weitere Monomere oder Prepolymere, Polymerisationsinhibitoren zum Stoppen der Polymerisation, Schutzlösungen für die mit dem Polymerisationssystem in Kontakt kommende Darmwand oder dergleichen enthalten. Drei bevorzugte Ausführungsformen werden nachstehend zur Illustration beschrieben.

### Beispiel 11 (nicht erfindungsgemäß): Die zweite Komponente enthält Polymerisationsinitiator

Ein polymerisierbare Zusammensetzung wie in Beispiel 7 beschrieben, d.h. mit einem bei Raumtemperatur startenden Initiator, z.B. dem Redoxinitiator Fe²⁺/H₂O₂, wird hier in Form eines Zweikomponenten-Systems eingesetzt, wobei Formulierung B den Initiator, gelöst in einer minimalen Menge Lösungsmittel, vorzugsweise Wasser, enthält und Formulierung A alle übrigen Bestandteile der Zusammensetzung enthält. Die beiden Formulierungen werden, ähnlich wie in den Beispielen 1 und 2 beschrieben, getrennt über zwei Kanäle des Endoskops zur Okklusionsstelle gepumpt und dort vermischt, wodurch die Polymerisation in Gang gesetzt wird. Der Vorteil gegenüber dem ansonsten identischen Einkomponenten-System besteht darin, dass Polymerisation im Kanal wirksam verhindert wird, wodurch auch reaktivere Initiatoren zum Einsatz kommen können, ohne die Zusammensetzung mittels Kühlung oder Inhibitoren hemmen zu müssen. Von der Erfindung umfasst sind freilich auch Fälle, in denen beide Formulierungen eine bestimmte Menge desselben Initiators oder auch zweier unterschiedlicher Initiatoren, z.B. eines Redox- und eines Photoinitiators, enthalten.

### Beispiel 12 (nicht erfindungsgemäß): Die zweite Komponente enthält Quellmittel

Eine ähnliche polymerisierbare Zusammensetzung wie in Beispiel 9 beschrieben, d.h. mit Photoinitiator, z.B. 2-Hydroxy-2-methyl-1-phenyl-1-propanon, aber einer minimalen Menge an Lösungsmittel, vorzugsweise einem Wasser/Glykolether-Gemisch, um alle Bestandteile zu lösen, wird als Formulierung A an die Okklusionsstelle gepumpt und dort mit UV-Strahlung polymerisiert. Gleichzeitig oder mit einer kurzen Verzögerung von wenigen Sekunden, z.B. 2 bis 3 Sekunden, wird als Formulierung B reines Wasser oder Wasser/Lösungsmittel-Gemisch, z.B. erneut Wasser/Glykolether, an die Okklusionsstelle gepumpt, um für ausreichende Quellung des gebildeten Polymerisats zu sorgen. Diese zweite Formulierung B kann entweder durch einen eigenen Kanal des Endoskops oder durch denselben wie zuvor die Formulierung A gepumpt werden, z.B. wenn im Endoskop nicht drei freie Kanäle zur Verfügung stehen. Der Vorteil eines solchen Systems besteht in der noch rascheren Polymerisation der Formulierung A als im Falle von Beispiel 9.

### Beispiel 13 (nicht erfindungsgemäß): Die zweite Komponente enthält ein Schutzmittel

Eine polymerisierbare Formulierung wie in einem der Beispiele 7 bis 11 beschrieben wird an die Okklusionsstelle gepumpt und dort polymerisiert. Davor wird allerdings der Bereich der Darmschleimhaut um die Okklusionsstelle, z.B. jeweils 5 bis 10 cm in oraler und aboraler Richtung, mit einem Film eines Schutzmittels ausgekleidet, der die Mukosa vor dem Kontakt mit dem Pfropfenmaterial, aber auch vor etwaiger Strahlung schützt. Zusätzlich dazu kann das Schutzmittel die Funktion eines Haftvermittlers erfüllen, um für bessere Haftung des Pfropfens an der Darmwand zu sorgen. Illustrative Beispiele sind wiederum die bereits erwähnten Gummen wie Tragant und Karaya, aber auch verschiedene andere schleimhautverträgliche Lösungen, Suspensionen und Gele, z.B. von Gelatine, Glykogen und anderen, vorzugsweise natürlichen, Polymeren. Eine solche vorhergehende Auskleidung der Mukosa kann freilich auch in Kombination mit beliebigen anderen Ausführungsbeispielen der Erfindung erfolgen.

Nachstehend werden verschiedene Varianten, den Pfropfen nach erfolgtem endoskopischem Eingriff wieder zu entfernen, beschrieben, wobei auch alle denkbaren Kombinationen der folgenden Beispiele sowie der zuvor allgemeiner beschriebenen Maßnahmen im Schutzumfang der Erfindung liegen.

### B) Verschiedene Arten der Aufhebung der Okklusion

### B1. Aufhebung der Okklusion auf mechanische Weise

### Beispiele 14 und 15 - Mechanische Ablösung des Pfropfens

Diese Beispiele illustrieren die bloße Ablösung des Polymerpfropfens von der Darmschleimhaut, wobei die anschließende endgültige Entfernung aus dem Darm prinzipiell auf beliebige Weise erfolgen kann.

### Beispiel 14: Wegziehen des Pfropfens

Ein Pfropfen mit ausreichender Festigkeit, z.B. ein zähes Gel, das durch Quellung oder Polymerisation von Gelatine bzw. modifizierter Gelatine erhalten wurde, oder ein mit einem Schaumstabilisator elastisch gemachter Polymerschaum, wird mittels eines Greifers am Ende des Endoskops in aboraler Richtung von der Okklusionsstelle weggezogen, so dass der Pfropfen nicht mehr an der Darmschleimhaut anhaftet, was vor allem in jenen Fällen ausreicht, bei denen die Okklusionsstelle vorbereitend mit einer Haftbeschichtung versehen worden war und das Pfropfenmaterial ohne diese Haftbeschichtung keine ausreichende Haftung an der Mukosa zeigt. Die anschließende Entfernung aus dem Darm erfolgt über den natürlichen Darmausgang durch den Druck nachkommender Exkremente und/oder mittels Auflösung durch das Darmsekret (optional unter zumindest teilweiser Resorption).

### Beispiel 15: Zufuhr von Scherspannung

In jenen erfindungsgemäß bevorzugten Fällen, wo der Pfropfen aufgrund von viskoelastischem bzw. thixotropem Verhalten an der Okklusionsstelle ausgebildet wurde, dringt der behandelnde Arzt zur Entfernung des Pfropfens von selbiger mit dem Endoskop, vorzugsweise mit einem entsprechenden Fortsatz an dessen Ende, in den Pfropfen ein und vollführt eine Rührbewegung, gegebenenfalls unter Ausübung eines leichten Drucks auf die Darmwand, um so eine Scherwirkung zu erzielen. Aufgrund der so zugeführten Scherspannung geht das viskoelastische/thixotrope Pfropfenmaterial wieder in einen fließfähigen Zustand über und rinnt zumindest teilweise von der Okklusionsstelle ab, wodurch die Okklusion aufgehoben wird. Das Abrinnen kann vom Arzt durch eine schabende Bewegung mit dem Endoskop unterstützt werden. Die endgültige Entfernung aus dem Darm kann wie in Beispiel 14 durch natürlichen Abgang und/oder durch zumindest teilweise Auflösung und gegebenenfalls Resorption erfolgen. Dieses Beispiel stellt genau genommen eine Kombination aus mechanischer und physikalischer Aufhebung der Okklusion dar.

### Beispiele 16 bis 19 - Mechanische Zerstörung des Pfropfens

Im Gegensatz zu den obigen Beispielen 14 und 15, wo die Strukturintegrität des Pfropfens durch mechanische Mittel entweder gar nicht oder zur Gänze aufgehoben wird, beschreiben die nächsten Beispiele Ausführungsformen unter teilweiser mechanischer Zerstörung der Pfropfenstruktur.

### Beispiel 16: Durchbohren des Pfropfens

Vor allem in Fällen, bei denen der Pfropfen aus einem Schaumkunststoff oder einem Gel besteht, der oder das aus im Darm abbaubarem Material, wie z.B. auf Polypeptid- oder Polysaccharid-Basis wie zuvor ausgeführt, aufgebaut ist, reicht eine Beschädigung der Okklusion mittels Durchbohrung des Pfropfens mit einem Dorn oder ähnlichen Fortsatz am Endoskopende aus, um die Okklusion aufzuheben und die Passage von Exkrementen wieder zu ermöglichen. Anschließend erfolgt ein allmählicher chemischer, z.B. enzymatischer, Abbau (oder Verdau) des Verschlussmaterials.

### Beispiel 17: Zerreißen des Pfropfens

In Fällen sehr guter Haftung eines im Vergleich zu Beispiel 14 strukturell instabileren, aber wie in Beispiel 15 im Darm abbaubaren Pfropfenmaterials an der Darmwand, z.B. eines Gels oder Schaums nach vorhergehender Aufbringung eines Haftmittels, kann ein Ergreifen des Pfropfens mit einem entsprechenden Werkzeug am Endoskop auch zum Zerreißen des Pfropfens führen. Das heißt, dass nach Aufhebung der Okklusion ein mehr oder weniger großer Anteil an der Darmwand haften bleibt und in der Folge chemisch abgebaut (z.B. verdaut) wird.

### Beispiel 18: Zerschneiden des Pfropfens

Ähnlich wie in Beispiel 15, allerdings besonders in jenen Fällen, bei denen zäheres und/oder elastisches, biologisch abbaubares Verschlussmaterial eingesetzt wurde, kann der Pfropfen durch Zerschneiden mit einem Skalpell oder ähnlichem Schneidwerkzeug am Endoskop zerstört werden. Moderne Endoskope sind mit solchen Werkzeugen standardmäßig ausrüstbar. Die Entfernung erfolgt vorzugsweise erneut mittels enzymatischen Abbaus.

### B2. Aufhebung der Okklusion auf physikalische Weise

### Beispiele 19 und 20 - Entfernung des Pfropfens durch Temperaturerhöhung

Ähnlich wie in den Beispielen 4 und 8 für die Ausbildung beschrieben kann auch die Entfernung des Pfropfens durch Bestrahlung mit Infrarotlicht und damit einhergehende Erwärmung vonstatten gehen.

### Beispiel 19: Auflösung des Pfropfens durch Sol/Gel-Übergang

Ein Pfropfen aus einer ähnlichen Zusammensetzung wie in Beispiel 1 beschrieben, d.h. ein Gel eines natürlichen Polymers wie Agar-Agar, wird mittels Bestrahlung mit Infrarotlicht über die Geltemperatur, z.B. über 45 °C, erwärmt, wodurch Verflüssigung eintritt und die Okklusion aufgehoben wird. Die endgültige Entfernung aus dem Darm kann wiederum durch natürlichen Abgang oder vorzugsweise enzymatischen Abbau erfolgen.

### Beispiel 20: Entfemung des Pfropfens durch thermische Schrumpfung

Ein durch Polymerisation erhaltener Pfropfen aus einem thermoresponsiven Hydrogel, wie z.B. Poly-N-isopropylacrylamid und Copolymeren davon, wird durch Bestrahlung mit Infrarotlicht erwärmt, wodurch sich das Hydrogel unter Wasserabgabe zusammenzieht und erheblich, z.B. um 20 bis 30 Prozent seines ursprünglichen Volumens, schrumpft, wonach eine Entfernung auf natürlichem Wege erfolgen kann, da für erneutes Quellen zu wenig Wasser zur Verfügung steht.

### Beispiele 21 bis 23 - Entfernung des Pfropfens durch Quellung oder Auflösung

Wie bereits zuvor erwähnt, kann die Struktur von Gelen durch Zusatz von weiterem Quellmittel, Elektrolytlösungen oder Lösungsmittel entscheidend verändert werden.

### Beispiel 21: Weitere Quellung

Auf einen Pfropfen aus einem unbegrenzt quellbaren Hydrogel, z.B. aus Gelatine, Agar-Agar oder anderen Polypeptiden oder Polysacchariden, beispielsweise wie in Beispiel 1 beschrieben, wird über einen Endoskopkanal weiteres Wasser gepumpt, vorzugsweise ein Mehrfaches des ursprünglichen Pfropfenvolumens, z.B. das Zweibis Dreifache, z.B. 200 bis 300 ml, wodurch das Gel schließlich die für das Gelieren erforderliche Konzentrationsgrenze, z.B. etwa 1 %, unterschreitet und sich daher verflüssigt.

### Beispiel 22: Schrumpfung

Ein Hydrogel, z.B. aus Gelatine wie in Beispiel 1, wird mit einer über den Endoskopkanal herbeigepumpten Elektrolytlösung, z.B. gesättigter oder physiologischer Kochsalzlösung, versetzt, wodurch Bindungsstellen im Polymer durch Salzionen abgesättigt werden und sich das Gel zusammenzieht und schrumpft und auf natürliche Weise und/oder durch Verdau und Resorption aus dem Darm entfernt wird.

### Beispiel 23: Zusatz eines Lösungsmittels

Ein Hydrogel, z.B. aus Gelatine wie in Beispiel 1, wird mit einem über den Endoskopkanal herbeigepumpten Lösungsmittel, im Fall von Gelatine z.B. mit stark verdünnter Essigsäure oder einem Glykol, wie z.B. Ethylenglykol, versetzt, wodurch die Gelatine in Lösung geht und abfließt.

### B3. Aufhebung der Okklusion durch Spaltung von chemischen Bindungen

### Beispiele 24 und 25 - Bindungsspaltung durch Bestrahlung

Die beiden folgenden Beispiele illustrieren kombinierte physikalisch-chemische Verfahren zur Spaltung von Bindungen im Pfropfenmaterial.

### Beispiel 24: Bestrahlung mit Infrarotlicht

Im Gegensatz zu Beispiel 20 ist im vorliegenden Fall durch die Infrarotstrahlung keine Erwärmung des Pfropfens als Ganzes und eine damit in Zusammenhang stehende Strukturänderung beabsichtigt, sondern die Spaltung temperaturempfindlicher chemischer Bindungen, wie z.B. von Azo-Bindungen, im Polymernetzwerk. Beispielsweise kann zur Herstellung der Okklusion eine Azo-Verbindung als bifunktionelles Monomer, d.h. als Vernetzer, mit einem monofunktionellen "Haupt"-Polymer, wie z.B. einem Vinylester-Monomer oder modifizierter Gelatine oder modifiziertem Agar-Agar, unter photochemischer Initiierung (z.B. mit Darocur® 1173) copolymerisiert werden. Wird der so gebildete Pfropfen nach erfolgtem endoskopischem Eingriff Infrarotstrahlung ausgesetzt, wird die Azo-Bindung unter N₂-Entwicklung gezielt an den Vernetzungsstellen gespalten, wodurch sich die Strukturintegrität des Pfropfens erheblich ändert.

Dies kann einerseits bewirken, dass das so gespaltene Material nicht mehr in ausreichendem Maße quellbar ist und sich mit anwesendem Wasser oder sonstigem Lösungsmittel verflüssigt oder zumindest erheblich schrumpft, oder andererseits, dass das Material gerade aufgrund des verringerten Vernetzungsgrads erhöhte Quellbarkeit aufweist, z.B. wenn vor der Spaltung ein sehr hoher Vernetzungsgrad und die daraus resultierende hohe Steifigkeit des Netzwerks das Quellvermögen stark eingeschränkt hatten. In beiden Fällen kann die anschließende Zufuhr von Quellmittel, z.B. Wasser, ähnlich wie in Beispiel 21, das In-Lösung-Gehen des gespaltenen Pfropfenmaterials auslösen. Letzteres kann, wie in einigen Beispielen zuvor beschrieben, enzymatisch weiter spaltbar und gegebenenfalls resorbierbar sein.

### Beispiel 25: Bestrahlung mit UVNIS-Licht

Hier werden Fälle beschrieben, bei denen ein mit UV- oder sichtbarem Licht spaltbarer Photosäurebildner, wie z.B. ein Nitrobenzylester oder ein Sulfonium- oder lodoniumsulfat-Salz, im Polymernetzwerk des Pfropfens enthalten ist. Dieser kann als Additiv darin vorliegen oder, bei entsprechender Modifikation, in die Polymerstruktur miteingebunden, d.h. copolymerisiert, sein. Als (sonstiges) Monomer kann wiederum z.B. ein Vinylester oder modifizierte(s) Gelatine oder Agar-Agar dienen, wobei jedoch ein Comonomer mit säurelabilen Bindungen, wie z.B. Acetal- oder Anhydrid-Bindungen, vorzugsweise ein als Vernetzer dienendes Comonomer, wie z.B. Methacrylsäureanhydrid oder vorzugsweise Divinyldicarbonat, enthalten sein muss. Die Polymerisation zur Herstellung des Pfropfens kann entweder thermisch oder mittels Redoxinitiator oder auch mittels eines Photoinitiators erfolgt sein, dessen Absorption bei deutlich unterschiedlicher Wellenlänge erfolgt als jene des Photosäurebildners.

Zur Zerstörung der Pfropfenstruktur nach dem endoskopischen Eingriff erfolgt eine Bestrahlung mit einer vom Photosäurebildner absorbierbaren Wellenlänge, wodurch Säure freigesetzt wird, die die Spaltung der säurelabilen Bindung bewirkt, was - je nach Vernetzungsgrad - ähnliche Konsequenzen haben kann, wie zuvor in Beispiel 24 beschrieben.

### Beispiele 26 bis 28 - Bindungsspaltung mit Säure, Base oder Enzymen

Anstatt im Pfropfenmaterial eine Säure durch Bestrahlung eines Photosäurebildners zu erzeugen, kann eine solche auch gezielt von außen zugeführt werden. Ähnliches gilt für andere Arten chemisch labiler Bindungen, wie z.B. mittels Basen oder enzymatisch spaltbare Bindungen.

### Beispiel 26: Zusatz von verdünnter Säure oder Base

Ein aus einem ähnlichen Material wie in Beispiel 25 bestehender Pfropfen wird mit einer wässrigen Lösung einer vorzugsweise schwachen, körperverträglichen Säure oder Base versetzt, d.h. die Lösung wird über den Endoskopkanal zur Okklusionsstelle gepumpt. Aufgrund der Einwirkung der Säure oder Base werden entsprechend labile Bindungen, vorzugsweise wiederum jene an Vernetzungsstellen, gespalten, was wiederum erhöhtes oder verringertes Quellvermögen oder auch verbesserte Zugänglichkeit für anschließende enzymatische Spaltung bewirken kann.

### Beispiel 27: Zusatz einer Enzymlösung

Anstelle oder zusätzlich zu einer Säure- oder Basenlösung wie in Beispiel 26 kann der Pfropfen mit einer Lösung eines das Polymernetzwerk angreifenden Enzyms versetzt werden. Im Falle eines Gelatine-Hydrogels kommen beispielsweise Peptidasen oder Peptidhydrolasen, wie z.B. Amino- oder Carboxypeptidasen, in Frage, für Agar-Agar- oder andere Polysaccharid-Gele hingegen entsprechende Glykosidasen, für Agar-Agar z.B. Galactosidasen, und für Glykoproteine und Proteoglykane Gemische beider Enzymarten. Vorzugsweise werden körpereigene Enzyme der jeweiligen Patientenspezies, d.h. insbesondere endogene Enzyme des menschlichen Körpers, eingesetzt.

### Beispiel 28: Spaltung des Pfropfens durch das Darmsekret

Wie bereits mehrmals erwähnt wurde, sind besonders bevorzugte Ausführungsformen der erfindungsgemäßen Zusammensetzung jene, die einen Pfropfen ergeben, der, gegebenenfalls nach vorheriger äußerer Einwirkung, in der natürlichen Umgebung des Darms, d.h. vor allem durch Einwirkung des Darmsekrets, abbaubar ist. Darunter fallen sowohl der pH-Wert des Darmsafts, durch den säurelabile, seltener auch basenlabile, Bindungen gespalten werden können, als auch die enzymatische Umgebung im jeweiligen Darmabschnitt, aufgrund derer der Pfropfen enzymatischen Abbau erfahren kann. Vorzugsweise wird das Pfropfenmaterial zu über die Darmwand resorbierbaren Bestandteilen der Nahrung des Patienten abgebaut.

Eine weitere Variante von natürlichem Abbau im Darm besteht jedoch auch in einem peripheren Abbau, d.h. in einer Spaltung und teilweisen Auflösung des Pfropfenmaterials an der Kontaktfläche zur Darmschleimhaut, wodurch einerseits die Haftung an der Darmwand aufgehoben wird, andererseits aber auch - während der anschließenden Weiterbeförderung aufgrund des Drucks nachkommender Exkremente in aboraler Richtung - das Volumen nach und nach in einem solchen Ausmaß verringert werden kann, dass keine Bedenken bezüglich des natürlichen Abgangs des Pfropfens durch den After bestehen. Als Pfropfenmaterialien kommen hierfür wiederum vor allem natürliche Polymere und Derivate davon, wie bereits mehrfach hierin beschrieben, in Frage.

### Beispiele 29 bis 31 - Modellversuche im Schweinedarm

Derzeit besonders bevorzugte Ausführungsformen der vorliegenden Erfindung umfassen gelierende Lösungen von Polypeptiden oder Polysacchariden, wie sie zuvor in den Beispielen 1 und 2 beschrieben wurden, da auf diese Weise die für den behandelten Patienten schonendste Art der Pfropfenbildung gewährleistet ist und gleichzeitig die Verwendung ausschließlich natürlicher Komponenten ermöglicht wird, vor allem solcher, die als Lebensmittelzusatzstoffe zugelassen sind. Insbesondere wird ein Zweikomponentensystem gemäß Beispiel 2 mit gesondertem Geliermittel bevorzugt, da so die Gelierung am gezieltesten bewirkt werden kann - sowohl hinsichtlich der Okklusionsstelle im Darm als auch des genauen Zeitpunkts der Pfropfenbildung.

Die Liste der einsetzbaren Polypeptide und Polysaccharide ist lang. Als Beispiele für in Lebensmitteln zugelassene Produkte seien daher als Polypeptide lediglich die tierischen Proteine Gelatine und Casein (Milchprotein) erwähnt und als Polysaccharide Alginate (E 400-405), Agar-Agar (E 406), Carrageen (E 407), Carubin (Johannisbrotkernmehl; E 410), Guar-Gummi (Guarkernmehl; E 412), Tragant (E 413), Gummi arabicum (E 414), Xanthan (E 415), Karaya (E 416) und Pektine (E 440), wobei in Klammern jeweils die Zulassungsnummer in der Europäischen Union angegeben ist. Diese Produkte sind physiologisch völlig unbedenklich, wobei Proteine beim Einsatz im Darm - je nach Verweilzeit - zum Teil abgebaut und resorbiert werden können, während die Polysaccharide vom Körper größtenteils nicht aufgenommen und ausgeschieden werden.

Es wurde eine Serie von Versuchen mit Gelatine sowie mit den aus Rotalgen extrahierten Polysacchariden Carrageen und Natriumalginat durchgeführt, wobei Lösungen, deren Konzentrationen jeweils auf Werte in der Nähe des Gelierpunkts eingestellt war, in von einem Schlachthof erhaltene Schweine-Naturdarm-Stücke (mit jeweils ca. 50 cm Länge) gefüllt und dort mit einer als Geliermittel dienenden zweiten Lösung vermischt wurden, um jeweils spontan einen Gelpfropfen zu bilden.

### Beispiel 29 (nicht erfindungsgemäß) - Natriumalginat

Wässrige Lösungen von Natriumalginat gelieren in Gegenwart von Calciumionen ebenfalls spontan. Die Calciumionen diffundieren dabei durch die spontan gebildete primäre Gelmembran nur bis zu einer definierten Gelschichtstärke. Unter dieser Gelschicht liegendes Natirumalginat wird nicht geliert und bleibt daher flüssig. Daher bildet sich bei geeigneter Eintragung des wässrigen Natriumalginats in eine wässrige Calciumionenlösung eine stabile Gelblase, die im Inneren aus ungeliertem Natriumalginat besteht. Die Stärke der Gelmembran wird durch die Anfangskonzentration der Natriumalginatlösung und die Konzentration der Calciumlösung bestimmt. Als Calciumsalz bietet sich Calciumchlorid an, das genau wie Natriumalginat selbst in pharmazeutischer Reinheit erhältlich ist.

Für 100%igen Umsatz bei der Vernetzungsreaktion reagieren 0,5 mol Calciumionen mit 1 Äquivalent Alginat. Generell kann daher eine ausreichend hohe einer Menge Lösung des Calciumsalzes in Wasser in ausreichend hoher Konzentration für zumindest 20%igen Umsatz über eine Kanüle eines Endoskops in den gewünschten Bereichs des Darms oberhalb des geplanten Stelle der Behandlung dosiert werden, um dort einen flüssigkeitsgefüllten Bereich auszubilden.

Beispielsweise wird eine Lösung von Natriumalginat mit einer bevorzugten Konzentration zwischen 0,1 und 5 Gew.-% in diesen flüssigkeitsgefüllten Bereich dosiert, wobei sich die aus einer Wand aus spontan geliertem Calciumalginat bestehende Gelblase ausbildet, die mit ungelierter Alginatsalz-Lösung gefüllt ist. Es wird dann so lange Alginatlösung in die Gelblase injiziert, bis diese die gewünschte Okklusion, d.h. den Gelpfropfen, an der gewünschten Stelle gebildet hat.

Die erzeugte Gelblase hat dann eine Größe, die zu einem kurzzeitigen Vollverschluss des Darms führt. Dabei tritt die Gelblase in Kontakt mit der umgebenden Darmwand und verschließt den Darm mechanisch. Die nowendige Größe der Gelblase und damit die notwendigen Mengen der beiden Lösungen richten sich nach der jeweiligen Situation im Zielbereich des Darms.

Zur Erleichterung der Ausbildung des flüssigkeitsgefüllten Raums kann die Calciumlösung mit geeigneten Verdickungsmittel, wie z.B. Stärkederivaten, hochviskos eingestellt werden. Zur Erhöhung der Haftung der Gelblase an der Darmwand kann der Alginatlösung darüber hinaus ein die Haftung an der Darmwand (Mucosa) förderndes Additiv, z.B. ein lösliches Protein wie etwa Casein, zugesetzt werden. Beide Lösungen können weiters mit einem Stabilisator, wie z.B. Kaliumsorbat, lagerstabilisiert werden.

Solange eine ausreichend hohe Calciumkonzentration in der Umgebung der gebildeten Gelblase vorherrscht, führt jede (mitunter irrtümliche, unerwünschte) Verletzung der Gelmembran der Gelblase zu einem Austritt von ungelierter Alginatlösung unter sofortiger Neubildung einer frischen Gelmembran, wodurch die Verletzung der Gelblase behoben wird. Die Kanüle des Endoskops ist am Austrittsende vorzugsweise so gestaltet, dass die Bildung der Gelblase begünstigt wird, d.h. beispielsweise trichterförmig erweitert oder gebördelt.

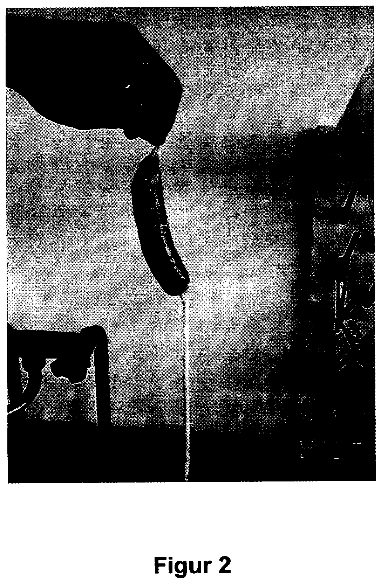

Konkret wurden 100 ml einer 1%igen wässrigen Lösung von Calciumchlorid-dihydrat (CaCl₂·2H₂O) in einen am Ende verknoteten Schweinedarm gefüllt. Anschließend wurden über eine in den so gebildeten Flüssigkeitskörper eingeführte Kanüle 50 ml einer 2%igen wässrigen Lösung von Natriumalginat in die Calciumchloridlösung eingeleitet. Beide Lösungen wiesen Raumtemperatur, d.h. 21 °C, auf, und die Alginatlösung wurde zur besseren Visualisierung dunkel (blau) eingefärbt. Das sich bereits zu Beginn der Einleitung an der Grenzfläche spontan bildende Calciumalginat bildete innerhalb des Flüssigkeitskörpers der Calciumchloridlösung eine feste Membran, die während der weiteren Zufuhr von Alginatlösung zu einer das Darmstück zur Gänze verschließenden Gelblase anschwoll. In Fig. 1 ist diese dunkel gefärbte Gelblase ebenso zu gut erkennen wie der darüber stehende Rest der Calciumchloridlösung. Diese Gelblase haftete beim anschließenden Umdrehen des Darms fest an der Darmwand an, wie in Fig. 2 dargestellt ist, und blieb auch während mehrstündiger Lagerung stabil.

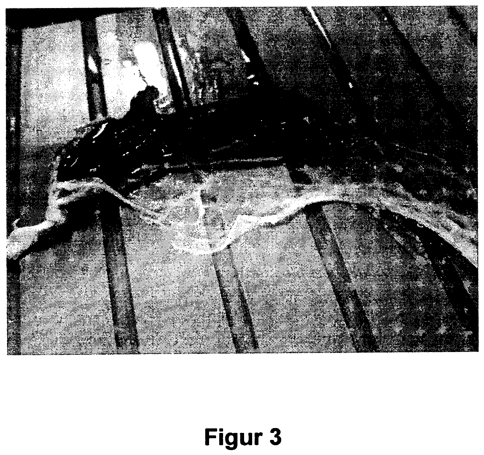

Zur Entfernung des so gebildeten Pfopfens wurde zunächst die ihn umgebende Calciumchloridlösung mit 200 ml entionisiertem Wasser weggespült. Danach wurde der Pfropfen mit einem Dorn durchbohrt, so dass die ungelierte Alginat-Lösung aus der Gelblase herausfloss. Die Gelmembran wurde danach mit den Fingern ergriffen und konnte leicht von der Darmwand abgelöst und aus dem Darmstück herausgezogen werden. Fig. 3 zeigt die entleerte Gelblase in einem der Länge nach aufgeschnittenen Darmstück, wobei ein Teil der Gelmembran bereits von Darm abgelöst ist, ohne zu zerreißen. Dieses Beispiel stellt somit eine Kombination der obigen Beispiele 2, 14 und 16 dar.

### Beispiel 30 (nicht erfindungsgemäß) - Carrageen

Carrageen ist in warmem Wasser leicht löslich, geliert aber beim Abkühlen ab einer Konzentration von etwa 2 Gew.-% zu Hydrogelen, weswegen es für Einkomponenten-Systeme wie in Beispiel 1 beschrieben gut geeignet wäre. Aber auch verdünntere, kalte Lösungen von Carrageen in Wasser werden, ähnlich wie Alginatlösungen, in Gegenwart von Calciumionen spontan zu Gelen vernetzt. Bei Carrageen kann eine solche Vernetzung darüber hinaus aber auch mit Kaliumionen erzielt werden.

Zur Verdeutlichung der allgemeinen Wirksamkeit des Prinzips der Pfropfenbildung durch Gelierung als Ausführungsform der vorliegenden Erfindung wurde bei der Verwendung von Carrageen der gegenüber dem obigen Beispiel 29 umgekehrte Weg gewählt und die Polysaccharidlösung im Schweinedarm vorgelegt, wonach die Metallionenlösung in diese injiziert wurde, um die Gelblase auszubilden. Diese Umkehrung der Reihenfolge (die natürlich auch im vorhergehenden Beispiel 29 funktioniert hätte) nutzt den Vorteil, dass auf knapp unterhalb des Gelierpunkts eingestellte Lösungen von Gelbildnern wie Polysacchariden und Proteinen ohnehin eine erhöhte Viskosität aufweisen können, weswegen mitunter kein zusätzlicher Verdicker erforderlich ist, um die zuerst in den Darm eingeleitete Lösung am Abfließen von der Okklusionsstelle zu hindern.

Konkret wurden 100 ml einer viskosen wässrigen Lösung von Carrageen mit einer Konzentration von 1 Gew.-% in einen Schweinedarm gefüllt, wonach über eine in das Zentrum des Flüssigkeitskörpers eingeführte Kanüle 50 ml einer 1%igen Lösung von Calciumchlorid-dihydrat (CaCl₂·2H₂O) in die Carrageenlösung eingeleitet wurden. Auch das Carrageen vernetzte an der Grenzfläche spontan unter Ausbildung eines weichen, elastischen Gels, das durch weitere Zufuhr von Calciumchloridlösung wiederum zu einer das Darmlumen ausfüllenden Gelblase anschwoll. Das Gel blieb ebenfalls beim Umdrehen des Darms fest an der Darmwand haften und während mehrstündiger Lagerung stabil.

Danach wurde in diesem Fall die unvernetzte Carrageenlösung mit entionisiertem Wasser weggespült, der gebildete Gelpfropfen mit den Fingern ergriffen und von der Darmwand abgezogen, wobei - aufgrund von höherer Haftung des Carrageen-Gels als im vorhergehenden Beispiel - die Gelmembran zerrissen wurde und unvernetzte Calciumchloridlösung ausfloss. Dieses Beispiel stellt somit eine Kombination der obigen Beispiele 2 und 17 dar.

### Beispiel 31 (nicht erfindungsgemäß): - Gelatine

Wässrige Lösungen von Gelatine mit einer Konzentration von etwa 1 Gew.-% oder darüber gelieren bei Temperaturen unter 35 °C spontan. Durch Erniedrigung der Löslichkeit mittels Erhöhung der lonenstärke der Gelatine-Lösung ist derselbe Gelierungseffekt erzielbar. Da Gelatine unbegrenzt quellbar ist, können solche Hydrogele durch Zufuhr von weiterem Wasser wieder in Lösung gebracht werden.

Daher wurden 100 ml einer auf Raumtemperatur (21 °C) abgekühlten, viskosen Lösung von Gelatine in Wasser mit einer Konzentration von etwa 0,9 Gew.-% in einen Schweinedarm gefüllt, wonach über eine in das Zentrum des Flüssigkeitskörpers eingeführte Kanüle 50 ml einer bei 35 °C bereiteten gesättigten Kochsalzlösung in die Gelatinelösung eingeleitet wurden. Die Gelatine gelierte spontan unter Ausbildung eines weichen Gels. Auch dieses Gel haftete beim Umdrehen des Darms fest an der Darmwand an und blieb bei mehrstündiger Lagerung stabil.

Der so gebildete Pfropfen wurde in der Folge durch langsames Einleiten von entionisiertem, auf 40 °C vorgewärmtem Wasser (insgesamt 750 ml) zu weiterer Quellung veranlasst, dabei allmählich wieder verflüssigt und aus dem Darm gespült. Dieses Beispiel stellt somit eine Kombination der obigen Beispiele 2 und 21 dar.

Als Ergebnis der obigen Modellversuche hat sich Beispiel 29 unter Verwendung von Natriumalginat als am vielversprechendsten herausgestellt. Die Anpassung der beiden Polysaccharidgele an das zu okkludierende Darmlumen durch Ausbildung einer Gelblase war - unter anderem wohl aufgrund des durch die Gelblase auf die Darmwand ausgeübten Drucks - einfacher möglich als mit Gelatine, wodurch auch eine dichtere Okklusion des Darmlumens erzielbar ist. Bei Verwendung von Natriumalginat konnte der Pfropfen zudem leichter von der Darmwand abgelöst werden als bei Verwendung von Carrageen, was wohl auf dessen natürliche Affinität zu Proteinen und die damit einhergehende stärkere Haftung an der Mucosa zurückzuführen ist. Somit stellt die Kombination Natriumalginat/Ca²⁺ die derzeit am meisten bevorzugte Ausführungsform der vorliegenden Erfindung dar.

Die vorliegende Erfindung stellt somit eine Reihe neuer Zusammensetzungen bereit, unter Verwendung derer auf vielfältige, aber durchwegs einfache und wirksame Weise eine vorübergehende Darmokklusion erzeugt werden kann, um einen endoskopischen Eingriff ungestört vornehmen zu können, wobei die Okklusion nach Beendigung des Eingriffs auf eine Vielzahl von Arten wieder entfernbar ist. Zur Erzeugung der Okklusion können herkömmliche Endoskope verwendet werden.

## Patentansprüche

1. Zusammensetzung zur Verwendung zur Erzeugung einer vorübergehenden Okklusion des Darms eines Säugetiers während eines endoskopischen Eingriffs, wobei die Zusammensetzung eine Lösung, Suspension oder Dispersion zumindest eines natürlichen oder künstlichen Polymers ist, die an einer gewünschten Stelle im Darm zu einem festen Pfropfen verfestigbar ist, dessen Struktur zur anschließenden, zumindest teilweisen Aufhebung der Okklusion veränderbar ist,
**dadurch gekennzeichnet, dass**
a) die Zusammensetzung thixotropes Verhalten zeigt;
b) die Zusammensetzung anfänglich fest, durch Rühren aber fließfähig machbar ist;
c) die Zusammensetzung im fließfähigen Zustand unter weiterer Energiezufuhr in Form von Schubspannung durch einen Kanal eines Endoskops an die gewünschte Stelle im Darm pumpbar ist; und
d) die Zusammensetzung durch Beendigung der Energiezufuhr zu dem festen Pfropfen verfestigbar ist.

2. Zusammensetzung zur Verwendung nach Anspruch 1, **dadurch gekennzeichnet, dass** die Zusammensetzung zu einem Pfropfen verfestigbar ist, dessen Struktur zur Aufhebung der Okklusion auf mechanische, physikalische und/oder chemische Weise zumindest teilweise zerstörbar ist.

3. Zusammensetzung zur Verwendung nach Anspruch 2, **dadurch gekennzeichnet, dass** die Struktur des Pfropfens durch Zufuhr mechanischer Energie zumindest teilweise zerstörbar ist.

4. Zusammensetzung zur Verwendung nach einem der vorangegangenen Ansprüche, **dadurch gekennzeichnet, dass** die Struktur des Pfropfens durch Quellung zumindest teilweise zerstörbar ist.

5. Zusammensetzung zur Verwendung nach einem der vorangegangenen Ansprüche, **dadurch gekennzeichnet, dass** die Struktur des Pfropfens durch Bestrahlung mit elektromagnetischen Strahlen zumindest teilweise zerstörbar ist.

6. Zusammensetzung zur Verwendung nach einem der Ansprüche 2 bis 5, **dadurch gekennzeichnet, dass** die Struktur des Pfropfens durch Spaltung chemischer Bindungen zumindest teilweise zerstörbar ist.

7. Zusammensetzung zur Verwendung nach Anspruch 6, **dadurch gekennzeichnet, dass** das Polymer labile Bindungen, ausgewählt aus aus hydrolyse-, licht- und temperaturempfindlichen Bindungen sowie enzymatisch spaltbaren Bindungen, enthält und die Struktur des Pfropfens durch Spaltung dieser Bindungen zumindest teilweise zerstörbar ist.

8. Zusammensetzung zur Verwendung nach Anspruch 7, **dadurch gekennzeichnet, dass** die labilen Bindungen aus der aus Acetal-, Ketal-, Ester-, Orthoester-, Azo-, Ether- und Anhydridbindungen bestehenden Gruppe ausgewählt sind.

9. Zusammensetzung zur Verwendung nach einem der Ansprüche 6 bis 8, **dadurch gekennzeichnet, dass** der feste Pfropfen durch Spaltung chemischer Bindungen zu Spaltprodukten umsetzbar ist, die durch Resorption über die Darmwand entfernbar sind.

10. Zusammensetzung zur Verwendung nach einem der vorangegangenen Ansprüche, **dadurch gekennzeichnet, dass** das Säugetier ein Mensch ist.

11. Zusammensetzung zur Verwendung nach einem der vorangegangenen Ansprüche, **dadurch gekennzeichnet, dass** die Zusammensetzung ein Thixotropiermittel enthält.

12. Zusammensetzung zur Verwendung nach Anspruch 11, **dadurch gekennzeichnet, dass** das Polymer selbst als Thixotropiermittel dient.

13. Zusammensetzung zur Verwendung nach Anspruch 11 oder 12, **dadurch gekennzeichnet, dass** das Thixotropiermittel ein oder mehrere Polyethylenglykole oder Polysaccharide umfasst.

14. Zusammensetzung zur Verwendung nach Anspruch 13, **dadurch gekennzeichnet, dass** das Thixotropiermittel aus Alginaten, Dextranen und Celluloseethern ausgewählt ist.

## Claims

1. Composition for the use of forming a temporary obstruction of the intestine of a mammal, the composition in the course of an endoscopic intervention, the composition being a flowable solution, suspension or dispersion of at least one natural or synthetic polymer, which is solidifyable to form a solid plug at a desired site of the intestine, the structure of said plug being subsequently changeable in order to at least partially remove said obstruction,
**characterized in that**
a) the composition shows thixotropic behavior;
b) initially, the composition is solid, but can be made flowable by stirring;
c) the composition, in its flowable state, is pumpable to the desired site of the intestine upon further energy supply in the form of shear stress; and
d) the composition is solidifiable to form said solid plug by stopping the supply of energy.

2. The composition for use according to claim 1, **characterized in that** the composition is solidifiable to form a plug, the structure of which is at least partially destroyable by mechanical, physical and/or chemical means to remove the obstruction.

3. The composition for use according to claim 2, **characterized in that** the structure of the plug is at least partially destroyable by supplying mechanical energy.

4. The composition for use according to any one of the preceding claims, **characterized in that** the structure of the plug is at least partially destroyable by swelling.

5. The composition for use according to any one of the preceding claims, **characterized in that** the structure of the plug is at least partially destroyable by irradiating electromagnetic radiation.

6. The composition for use according to any one of the claims 2 to 5, **characterized in that** the structure of the plug is at least partially destroyable by cleaving chemical bonds.

7. The composition for use according to claim 6, **characterized in that** the polymer contains labile bonds, selected from hydrolysis-, light- and temperature-sensitive bonds as well as enzymatically cleavable bonds, and the structure of the plug is at least partially destroyable by cleaving said bonds.

8. The composition for use according to claim 7, **characterized in that** the labile bonds are selected from the group consisting of acetal, ketal, ester, ortho-ester, azo, ether and anhydride bonds.

9. The composition for use according to any one of the claims 6 to 8, **characterized in that**, by cleaving chemical bonds, the solid plug is convertable into cleavage products which are removable by being resorbed by the intestinal wall.

10. The composition for use according to any one of the preceding claims, **characterized in that** the mammal is a human being.

11. The composition for use according to any one of the preceding claims, **characterized in that** the composition contains a thixotropic agent.

12. The composition for use according to claim 11, **characterized in that** the polymer itself serves as the thixotropic agent.

13. The composition for use according to claim 11 or 12, **characterized in that** the thixotropic agent comprises one or more polyethylene glycols or polysaccharides.

14. The composition for use according to claim 13, **characterized in that** the thixotropic agent is selected from alginates, dextranes and cellulosic ethers.

## Revendications

1. Composition en vue d'une utilisation dans la production d'une occlusion intestinale provisoire dans un mammifère pendant une intervention endoscopique, la composition étant une solution, suspension ou dispersion d'au moins un polymère naturel ou synthétique, qui peut être solidifiée à une position désirée dans l'intestin pour donner un bouchon solide, dont la structure est changeable pour, ensuite, éliminer l'occlusion, au moins en partie,
**caractérisée en ce que**
a) la composition a un comportement thixotrope;
b) la composition est d'abord solide, mais peut être liquéfiée en étant remuée;
c) dans son état liquide, la composition peut être pompée à travers un canal d'un endoscope jusqu'à la position désirée dans l'intestin moyennant l'apport d'énergie supplémentaire sous forme de tension de cisaillement;
d) la composition peut être solidifiée pour donner ledit bouchon solide moyennant l'interruption de l'apport d'énergie;

2. Composition en vue d'une utilisation selon la revendication 1, **caractérisée en ce que** la composition peut être solidifiée pour donner un bouchon, dont la structure est destructible, au moins en partie, de façon mécanique, physique et/ou chimique pour éliminer l'occlusion.

3. Composition en vue d'une utilisation selon la revendication 2, **caractérisée en ce que** la structure du bouchon est destructible, au moins en partie, par l'apport d'énergie mécanique.

4. Composition en vue d'une utilisation selon l'une quelconque des revendications précédentes, **caractérisée en ce que** la structure du bouchon est destructible, au moins en partie, par gonflement.

5. Composition en vue d'une utilisation selon l'une quelconque des revendications précédentes, **caractérisée en ce que** la structure du bouchon est destructible, au moins en partie, par irradiation avec des rayons électromagnétiques.

6. Composition en vue d'une utilisation selon l'une quelconque des revendications 2 à 5, **caractérisée en ce que** la structure du bouchon est destructible, au moins en partie, par clivage de liaisons chimiques.

7. Composition en vue d'une utilisation selon la revendication 6, **caractérisée en ce que** le polymère contient des liaisons labiles, sélectionnées parmi des liaisons sensibles à l'hydrolyse, à la lumière et à la température, ainsi que des liaisons clivables de manière enzymatique, et **en ce que** la structure du bouchon est destructible, au moins en partie, par clivage de ces liaisons.

8. Composition en vue d'une utilisation selon la revendication 7, **caractérisée en ce que** les liaisons labiles sont sélectionnées parmi le groupe composé de liaisons acétal, cétal, orthoester, azo, éther et anhydrite.

9. Composition en vue d'une utilisation selon l'une quelconque des revendications 6 à 8, **caractérisée en ce que** le bouchon solide est transformable, par le clivage de liaisons chimiques, en produits de clivage etant éliminable par résorption à travers la paroi intestinale.

10. Composition en vue d'une utilisation selon l'une quelconque des revendications précédentes, **caractérisée en ce que** le mammifère est un être humain.

11. Composition en vue d'une utilisation selon l'une quelconque des revendications précédentes, **caractérisée en ce que** la composition contient un agent thixotrope.

12. Composition en vue d'une utilisation selon la revendication 11, **caractérisée en ce que** le polymère même sert dudit agent thixotrope.

13. Composition en vue d'une utilisation selon les revendications 11 ou 12, **caractérisée en ce que** l'agent thixotrope comprend un ou plusieurs polyéthylène glycols ou des polysaccharides.

14. Composition en vue d'une utilisation selon la revendication 13, **caractérisée en ce que** l'agent thixotrope est sélectionné parmi des alginates, des dextranes et des éthers de cellulose.
